# EUROPEAN PATENT APPLICATION

(11) **EP 4 656 186 A1**
(43) Date of publication of application: **03.12.2025**
(21) Application number: 24771137.7
(22) Date of filing: 08.03.2024
(51) Int. Cl.: A61K 9/16, A61K 31/519

(54) **NOVEL SOLID DISPERSION AND METHOD FOR PREPARING SAME**

(30) Priority: 10.03.2023 KR 20230032080
(71) Applicant: ST Pharm Co., Ltd., Siheung-si, Gyeonggi-do 15086 (KR)
(72) Inventor: KIM, Kyungjin, Seoul 06170 (KR); KIM, Uk-Il, Ansan-si, Gyeonggi-do 15610 (KR)
(74) Representative: Fuchs Patentanwälte Partnerschaft mbB
(86) International application number: PCT/KR2024/003042
(87) International publication number: WO 2024/191137

(57) **Abstract**

The present invention relates to a solid dispersion of a triazolopyrimidinone derivative exhibiting tankyrase inhibitory activity, and a method for preparing the same. The solid dispersion of the triazolopyrimidinone derivative represented by Chemical Formula I according to the present invention has high solubility and excellent bioavailability, and thus can be usefully employed as a pharmaceutical raw material.

## Description

### [Technical Field]

The present disclosure relates to a solid dispersion of a compound represented by Chemical Formula I, which has significantly improved solubility and bioavailability, a pharmaceutical formulation comprising the same, and a method for preparing the same:

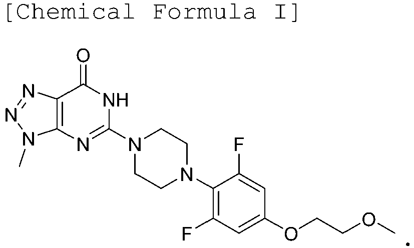

### [Background Art]

Tankyrase belongs to the poly (ADP-ribose) polymerase (PARP) protein family which consists of 17 members sharing a catalytic PARP domain. Recently, it has been reported that intracellular axin levels are affected by PARP enzyme family members, tankyrase-1 and tankyrase-2 (also known as PARP5a and PARP5b, respectively) (Huang et al., 2009, Nature, 461 (7264): 614-620).

Inhibitors of tankyrase-1 and tankyrase-2 are known to have therapeutic potential for various cancer diseases, including solid tumors such as colorectal carcinoma, colon cancer, gastric cancer, hepatocellular carcinoma, breast cancer, medulloblastoma, melanoma, non-small cell lung cancer, pancreas adenocarcinoma, and prostate cancer. In addition, the inhibitors of tankyrase-1 and tankyrase-2 have therapeutic potential for other diseases in addition to the cancer diseases, including osteoporosis, osteoarthritis, polycystic kidney disease, pulmonary fibrosis, diabetes, schizophrenia, vascular disease, cardiac disease, non-oncogenic proliferative diseases, and neurodegenerative diseases such as Alzheimer's disease.

As described above, the demand for novel therapeutic agents capable of being used for cancer and hyperproliferative conditions continues, and efforts are being made to develop novel pharmaceutical compounds capable of selectively inhibiting tankyrase enzymes. In particular, triazolopyrimidinone derivatives of the following Chemical Formula I are known as selective tankyrase inhibitors (International Patent Publication No. WO2016/006974), and are being developed as therapeutic agents for colorectal cancer in patients with a colorectal cancer-inducing gene (KRAS: Kirsten rat sarcoma virus) mutant genotype or Erbitux non-responsive patients.

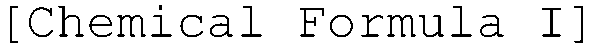

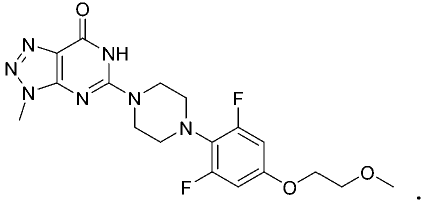

However, the triazolopyrimidinone derivative of Chemical Formula I is a crystalline material belonging to Biopharmaceuticals Classification System (BCS) class II, and has physicochemical properties of high permeability and very low solubility.

Therefore, the development of a novel formulation is required to address the issue of low solubility and enhance the bioavailability of the triazolopyrimidinone derivative of Chemical Formula I.

### [Disclosure]

### [Technical Problem]

An object of the present disclosure is to provide a novel solid dispersion of a triazolopyrimidinone derivative of Chemical Formula I having significantly improved solubility and bioavailability.

In addition, another object of the present disclosure is to provide a pharmaceutical formulation comprising the solid dispersion.

Further, still another object of the present disclosure is to provide a novel method for preparing the solid dispersion.

### [Technical Solution]

In order to achieve the above objects, the present inventors have studied and made efforts, and as a result, developed a novel solid dispersion including a compound represented by the following Chemical Formula I and a method for preparing the same, and confirmed that the solid dispersion has significantly improved solubility and bioavailability, thereby completing the present disclosure:

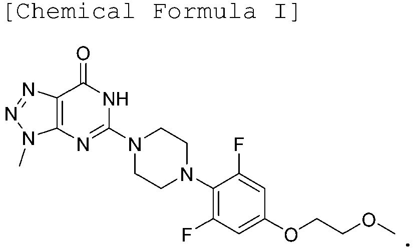

### Solid Dispersion of Compound Represented by Chemical Formula I

The solid dispersion of the compound represented by Chemical Formula I according to the present disclosure is characterized by significantly improved solubility and bioavailability compared to the compound represented by Chemical Formula I.

The solid dispersion of the compound represented by Chemical Formula I of the present disclosure includes the compound represented by Chemical Formula I and a pH-dependent polymer.

Specifically, the present disclosure provides a solid dispersion comprising a compound represented by the following Chemical Formula I and a pH-dependent polymer:

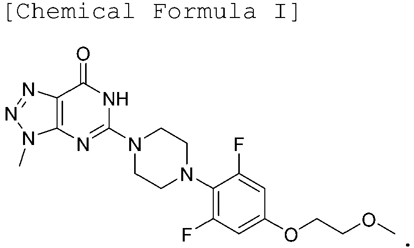

In the present disclosure, the pH-dependent polymer refers to a polymer whose charge state varies under pH conditions typically found in the gastrointestinal tract, specifically within the pH range of 1 to 8. The pH-dependent polymer may be, for example, a polymer material having a functional group whose charge state varies depending on pH conditions, including a basic functional group such as an amino group, or an acidic functional group such as a carboxylic acid.

The pH-dependent polymer of the present disclosure may be included alone or in plurality in the solid dispersion according to the present disclosure.

According to an embodiment of the present disclosure, the pH-dependent polymer may be a methacrylic acid-methyl methacrylate copolymer, a methacrylic acid-ethyl acrylate copolymer, hydroxypropyl methylcellulose phthalate (HPMCP), hydroxypropyl methylcellulose acetate succinate (HPMCAS), hydroxypropyl methylcellulose acetate phthalate (HPMCAP), carboxymethyl ethylcellulose (CMEC), cellulose acetate phthalate (CAP) or a combination thereof.

Specifically, the methacrylic acid-methyl methacrylate copolymer may be Eudragit L100 or Eudragit S100, and the methacrylic acid-ethyl acrylate copolymer may be Eudragit L100-55 or Eudragit L30D-55.

Preferably, in the solid dispersion of the present disclosure, the pH-dependent polymer may be hydroxypropyl methylcellulose acetate succinate (HPMCAS).

According to an embodiment of the present disclosure, a weight ratio of the compound represented by Chemical Formula I and the pH-dependent polymer may be 1 : 1 to 1 : 10. The weight ratio may be preferably 1 : 2 to 1 : 4, and more preferably 1 : 4.

Preferably, in the solid dispersion of the present disclosure, the weight ratio of the compound represented by Chemical Formula I and hydroxypropyl methylcellulose acetate succinate (HPMCAS) may be 1 : 1 to 1 : 10, more preferably 1 : 2 to 1 : 4.

According to an embodiment of the present disclosure, the weight ratio of the compound represented by Chemical Formula I and hydroxypropyl methylcellulose acetate succinate (HPMCAS) may be 1 : 4.

According to an embodiment of the present disclosure, a loading percentage of the compound represented by Chemical Formula I may be 1 to 50 w/w%. The loading percentage may preferably be 10 to 40 w/w%.

In the solid dispersion including the compound represented by Chemical Formula I and the pH-dependent polymer according to the present disclosure, the compound represented by Chemical Formula I in the solid dispersion may be amorphous.

Further, in the solid dispersion including the compound represented by Chemical Formula I and the pH-dependent polymer according to the present disclosure, the compound represented by Chemical Formula I in the solid dispersion may be partially amorphous or partially crystalline.

### Pharmaceutical Formulation Comprising Solid Dispersion of Compound Represented by Chemical Formula I

The present disclosure provides a pharmaceutical formulation comprising a solid dispersion including the compound represented by Chemical Formula I and a pH-dependent polymer.

In the present disclosure, the pharmaceutical formulation may further comprise a pharmaceutically acceptable additive within a range that does not impair the effects of the present disclosure. The pharmaceutically acceptable additive may be an excipient, a binder, a disintegrant, a lubricant, a coating agent, a solubilizer, a stabilizer, or a combination thereof.

The excipient may be any excipient known in the art, and for example, may be selected from the group consisting of lactose, microcrystalline cellulose, low-substituted hydroxypropylcellulose, calcium phosphate, light anhydrous silicic acid, colloidal silicon dioxide, magnesium aluminate metasilicate, pregelatinized starch, corn starch, potato starch, white sugar, mannitol, dextrin, precipitated calcium carbonate, and any combination thereof.

The disintegrant may be any disintegrant known in the art, and for example, may be selected from the group consisting of low-substituted hydroxypropylcellulose, crospovidone, sodium starch glycolate, polyvinylpyrrolidone, croscarmellose sodium, pregelatinized starch, starch, alginic acid, sodium alginate, and a combination thereof.

The binder may be any binder known in the art, and may be, for example, any one or more selected from the group consisting of polyvinylpyrrolidone, povidone, and copovidone.

The lubricant may be any lubricant known in the art, and for example, may be selected from the group consisting of stearic acid, stearic acid metal salts, talc, sucrose fatty acid esters, hydrogenated vegetable oils, high melting point waxes, glyceryl fatty acid esters, glyceryl dibehenate, and any combination thereof.

The coating agent may be any coating agent known in the art, and may be selected from the group consisting of polyvinyl alcohol, talc, hydroxypropylmethylcellulose, ethylcellulose, polyvinyl acetate, titanium dioxide, iron oxide, and a combination thereof.

The solubilizer may be any solubilizer known in the art, and may be, for example, any one or more selected from the group consisting of sodium lauryl sulfate and polysorbate.

The stabilizer may be any stabilizer known in the art, and may be, for example, any one or more selected from the group consisting of citric acid, fumaric acid, hydrochloric acid, benzoic acid, sodium hydrogen carbonate, sodium hydroxide, calcium carbonate, and meglumine.

A method of formulating the pharmaceutical formulation of the present disclosure may be a formulation method commonly used in the art, and the pharmaceutical formulation may be administered orally or parenterally. The form of the pharmaceutical formulation including the solid dispersion of the present disclosure may be, for example, granules, powders, tablets, coated tablets, capsules, syrups, suspensions, emulsions, ointments, creams, gels, drops, aerosols, or injectable solutions, but is not limited thereto. In addition, the daily effective dose of the pharmaceutical formulation may be appropriately changed depending on the patient's age, weight, sex, dosage form, health condition, or severity of the disease. While not particularly limited in the present disclosure, the dose may range from 1 mg to 1000 mg for an adult and may be administered once daily or in divided doses multiple times per day.

### Method for Preparing Solid Dispersion of Compound Represented by Chemical Formula I

The present disclosure provides a method for preparing a solid dispersion of the compound represented by Chemical Formula I.

The solid dispersions of the present disclosure may be prepared using methods for preparing a solid dispersion well known in the art and may be prepared by any known means including, for example, spray drying, hot melt extrusion or precipitation from solution by anti-solvent addition.

Preferably, the solid dispersion of the present disclosure may be prepared using a Micro-precipitated bulk powder (MBP) technique.

Specifically, the preparation method includes the following steps (S-1) to (S-3):
(S-1) dissolving a compound represented by the following Chemical Formula I and a pH-dependent polymer in an organic solvent to prepare a solution;
(S-2) adding the solution dropwise to an acidic anti-solvent or distilled water to precipitate a solid; and
(S-3) filtering the solid, followed by washing with an acidic anti-solvent or distilled water, and then drying;

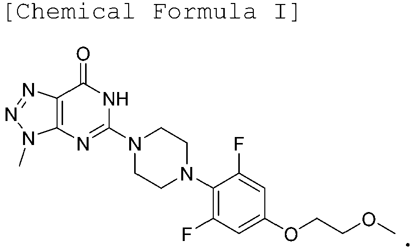

The description of the solid dispersion of the present disclosure may be applied to the details of the method for preparing the solid dispersion as it is. Specifically, the compound represented by Chemical Formula I, the pH-dependent polymer, and the weight ratio thereof are as described above.

Hereinafter, steps (S-1) to (S-3) will be described in detail.

### Step (S-1)

Step (S-1) of the present disclosure is a step of dissolving the compound represented by Chemical Formula I and the pH-dependent polymer in an organic solvent to prepare a uniform solution.

According to an embodiment of the present disclosure, the organic solvent may be dimethylformamide (DMF), dimethylacetamide (DMA), dichloromethane (DCM), chloroform or N-methylpyrrolidone (NMP). Preferably, dimethylacetamide (DMA) may be used as the organic solvent.

According to an embodiment of the present disclosure, the percent concentration of the solution may be 1 to 50 w/v%. Preferably, a solution of 10 to 30 w/v% may be prepared and used, and more preferably, a solution of 10 w/v% may be prepared and used.

### Step (S-2)

Step (S-2) of the present disclosure is a step of slowly adding the solution prepared in Step (S-1) dropwise to an anti-solvent to precipitate a solid.

In the present disclosure, the anti-solvent refers to a solvent capable of precipitating a solute by lowering the solubility of a solute dissolved in a solution and inducing the solution in a supersaturated state. Specifically, the anti-solvent of Step (S-2) of the present disclosure may be an acidic anti-solvent or distilled water.

According to an embodiment of the present disclosure, the acidic anti-solvent may be an aqueous solution of hydrochloric acid, sulfuric acid, phosphoric acid, nitric acid, acetic acid, citric acid or lactic acid.

Preferably, the anti-solvent of Step (S-2) may be an aqueous hydrochloric acid solution or distilled water, more preferably 0.01 N aqueous hydrochloric acid solution or distilled water.

According to an embodiment of the present disclosure, a temperature for dropwise addition in Step (S-2) may be 0 to 10°C. The temperature for dropwise addition means that the temperature of the acidic anti-solvent or distilled water is maintained at 0 to 10°C from the start of the dropwise addition until its completely termination. The temperature for dropwise addition may be preferably 0 to 5°C, more preferably 0°C.

According to an embodiment of the present disclosure, Step (S-2) may further comprise a step of stirring at room temperature after dropwise addition. Specifically, the stirring step is a step of stirring the mixture, which is obtained after the dropwise addition is completed, at 15 to 25°C. Preferably, the stirring may be performed for 30 minutes.

### Step (S-3)

Step (S-3) of the present disclosure is a step of filtering the solid precipitated in Step (S-2), followed by washing with an acidic anti-solvent or distilled water, and then drying.

According to an embodiment of the present disclosure, the acidic anti-solvent may be an aqueous solution of hydrochloric acid, sulfuric acid, phosphoric acid, nitric acid, acetic acid, citric acid or lactic acid. Preferably, an aqueous hydrochloric acid solution may be used, and more preferably, a 0.01 N aqueous hydrochloric acid solution may be used.

In Step (S-3), the washing may be performed one or more times, and for example, the washing may be performed once with a 0.01 N aqueous hydrochloric acid solution or distilled water, and then the washing may be further performed twice with distilled water.

In addition, in the preparation method of the present disclosure, stirring, filtration, washing, and drying may be performed without limitation using methods known in the art.

Further, the present disclosure provides a solid dispersion of the compound represented by Chemical Formula I prepared according to the preparation method described in steps (S-1) to (S-3).

### [Advantageous Effects]

The solid dispersion of the compound represented by Chemical Formula I according to the present disclosure has high solubility and excellent bioavailability, and thus can be usefully employed as a pharmaceutical raw material.

### [Best Mode]

Hereinafter, the present disclosure will be described in detail with reference to Examples and Experimental Examples. However, the following Examples and Experimental Examples are only illustrative of the present disclosure, and the scope of the present disclosure is not limited thereto.

### Example 1: Preparation of Solid Dispersion of Compound Represented by Chemical Formula I

1 g of the compound represented by Chemical Formula I and 4 g of HPMCAS were mixed and completely dissolved in 50 ml of DMA. After cooling 500 ml of 0.01 N HCl or distilled water to 0°C, the solution prepared above was slowly added dropwise. Then, the resulting mixture was stirred at room temperature for 30 minutes, filtered, and washed once with 0.01 N HCl or distilled water. Thereafter, the resulting mixture was washed twice more with distilled water and vacuum dried to obtain a solid dispersion of the compound represented by Chemical Formula I.

### Examples 2 to 5: Preparation of Solid Dispersions of Compounds Represented by Chemical Formula I

Each solid dispersion was prepared in the same manner as in Example 1, except that Eudragit L100-55, Eudragit L100, Eudragit S100, or HPMCP was used instead of HPMCAS.

### Experimental Example 1: Confirmation of Drug Load and Purity of Examples 1 to 5

The drug load and purity of the solid dispersions prepared in Examples 1 to 5 (the compounds represented by Chemical Formula I) were confirmed by HPLC (high performance liquid chromatography, manufacturer: Agilent Technologies, model name: 1260 Infinity II), and the results are shown in Table 1.

### Experimental Example 2: Confirmation of Solubility of Examples 1 to 5 in FaSSIF

The solubility of the solid dispersions prepared in Examples 1 to 5 in fasted state simulated intestinal fluid (FaSSIF) was confirmed, and the results are shown in Table 1.

**[Table 1]**

| | Drug load (wt%) | Purity | FaSSIF Solubility (After 4 hours at room temperature, µg/mL) |
|---|---|---|---|
| Compound of Chemical Formula I | 100% | 99.04% | 1.89 |
| Example 1 | 19.2% | 98.54% | 18.91 |
| Example 2 | 14.3% | 98.68% | 13.74 |
| Example 3 | 13.05% | 98.91% | 3.66 |
| Example 4 | 15.04% | 98.74% | 13.40 |
| Example 5 | 14.38% | 98.83% | 9.68 |

As a result, as shown in Table 1, it was confirmed that all the solid dispersions of Examples had increased solubility compared to the compound represented by Chemical Formula I, and in particular, the solid dispersion of Example 1 including HPMCAS was confirmed to have a solubility that was approximately 10 times higher than that of the compound represented by Chemical Formula I.

### Examples 6 to 7: Preparation of Solid Dispersions of Compounds Represented by Chemical Formula I

Solid dispersions were prepared by mixing HPMCAS, which is a pH-dependent polymer of the solid dispersion exhibiting the best solubility, with a drug (a compound represented by Chemical Formula I) in various ratios.

Specifically, each solid dispersion was prepared in the same manner as in Example 1, except that 2 g or 2.5 g of HPMCAS was used relative to 1 g of the compound represented by Formula I.

### Experimental Example 3: Confirmation of Drug Load and Purity of Examples 6 to 7

The load and purity of the solid dispersions prepared in Examples 6 to 7 (the compounds represented by Chemical Formula I) were confirmed in the same manner as in Experimental Example 1, and the results are shown in Table 2.

### Experimental Example 4: Confirmation of Solubility of Examples 6 to 7 in FaSSIF

The solubility of the solid dispersions prepared in Examples 6 to 7 in fasted state simulated intestinal fluid (FaSSIF) was confirmed in the same manner as in Experimental Example 2, and the results are shown in Table 2.

**[Table 2]**

| | Drug load (wt%) | Purity | FaSSIF Solubility (After 4 hours at room temperature, µg/mL) |
|---|---|---|---|
| Compound of Chemical Formula I | 100% | 99.04% | 1.66 |
| Example 6 | 31.3% | 98.54% | 16.57 |
| Example 7 | 26.6% | 99.29% | 16.21 |
| Example 1 | 19.2% | 98.54% | 18.91 |

As a result, as shown in Table 2, it was confirmed that all the solid dispersions of Examples had increased solubility compared to the compound represented by Chemical Formula I, and in particular, the solid dispersion of Example 1 including the compound represented by Chemical Formula I and HPMCAS in a weight ratio of 1 : 4 was confirmed to have a solubility that was approximately 11 times higher than that of the compound represented by Chemical Formula I.

### Experimental Example 5: Confirmation of Bioavailability of Examples 1 and 6

In order to confirm the bioavailability of the solid dispersion of Example 1, which showed the best solubility, and the solid dispersion of Example 6, a pharmacokinetic test (*in vivo*) was performed in a mouse model.

Specifically, the drug was administered orally to rats, and blood was collected from the jugular vein at a predetermined time. The collected blood was centrifuged to separate plasma, pretreated, and then the concentration was analyzed by LC-MS/MS. Non-compartmental pharmacokinetic parameters were calculated using WinNonlin (Phoenix^{™}, version 6.1) from the analyzed blood concentration-time data of the drug, and the results are shown in Table 3.

**[Table 3]**

| | Dose (mg/kg) | Tₘₐₓ (hr) | T_{1/2} (hr) | Cₘₐₓ (ng/mL) | AUCₗₐₛₜ (hr*ng/mL) | Cl_F_obs (mL/hr/kg) | AUCₗₐₛₜ Fold change |
|---|---|---|---|---|---|---|---|
| Compound of Chemical Formula I | 10 | 1 | 5.39 | 1,021 | 5,458 | 1, 788 | 1.00 |
| Example 6 | 10 | 0.5 | 6.11 | 2,274 | 15, 139 | 685 | 2.77 |
| Example 1 | 10 | 0.5 | 5.30 | 111,880 | 38, 879 | 249 | 7.12 |

As a result, as shown in Table 3, it was confirmed that the solid dispersions of Examples 1 and 6 all exhibited excellent bioavailability with high Cₘₐₓ and AUC values compared to the compound represented by Chemical Formula I. In particular, the solid dispersion of Example 1 including the compound represented by Chemical Formula I and HPMCAS in a weight ratio of 1 : 4 was confirmed to have the AUC that was approximately 7 times higher than that of the compound represented by Chemical Formula I.

Although the present disclosure has been described in detail through preferred embodiments, the scope of the present disclosure is not limited to a specific embodiment and should be interpreted by the appended claims. In addition, it should be understood by those skilled in the art that many modifications and variations are possible without departing from the scope of the present disclosure.

## Claims

1. A solid dispersion comprising a compound represented by the following Chemical Formula I and a pH-dependent polymer:

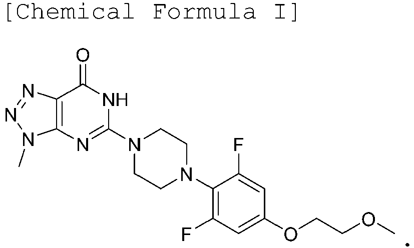

2. The solid dispersion of claim 1, wherein the pH-dependent polymer is a methacrylic acid-methyl methacrylate copolymer, a methacrylic acid-ethyl acrylate copolymer, hydroxypropyl methylcellulose phthalate (HPMCP), hydroxypropyl methylcellulose acetate succinate (HPMCAS), hydroxypropyl methylcellulose acetate phthalate (HPMCAP), carboxymethyl ethylcellulose (CMEC), cellulose acetate phthalate (CAP) or a combination thereof.

3. The solid dispersion of claim 1, wherein a weight ratio of the compound represented by Chemical Formula I and the pH-dependent polymer is 1 : 1 to 1 : 10.

4. The solid dispersion of claim 1, wherein a weight ratio of the compound represented by Chemical Formula I and the pH-dependent polymer is 1 : 2 to 1 : 4.

5. The solid dispersion of claim 1, wherein a loading percentage of the compound represented by Chemical Formula I is 1 to 50 w/w%.

6. A pharmaceutical formulation comprising the solid dispersion according to any one of claims 1 to 5.

7. A method for preparing a solid dispersion of the compound represented by Chemical Formula I according to any one of claims 1 to 5, the method comprising:
(S-1) dissolving a compound represented by the following Chemical Formula I and a pH-dependent polymer in an organic solvent to prepare a solution;
(S-2) adding the solution dropwise to an acidic anti-solvent or distilled water to precipitate a solid; and
(S-3) filtering the solid, followed by washing with an acidic anti-solvent or distilled water, and then drying:

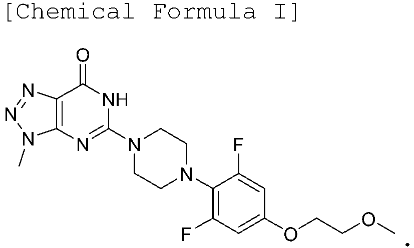

8. The method of claim 7, wherein the organic solvent is dimethylformamide (DMF), dimethylacetamide (DMA), dichloromethane (DCM), chloroform or N-methylpyrrolidone (NMP).

9. The method of claim 7, wherein a percent concentration of the solution is 1 to 50 w/v%.

10. The method of claim 7, wherein the acidic anti-solvent is an aqueous solution of hydrochloric acid, sulfuric acid, phosphoric acid, nitric acid, acetic acid, citric acid or lactic acid.

11. The method of claim 7, wherein a temperature for dropwise addition in Step (S-2) is 0 to 10°C.

12. The method of claim 7, wherein Step (S-2) further comprises stirring at room temperature after dropwise addition.
